# EUROPÄISCHE PATENTSCHRIFT

(11) **EP 4 317 398 B1**
(45) Veröffentlichungstag und Bekanntmachung des Hinweises auf die Patenterteilung: **30.04.2025**
(21) Anmeldenummer: 22188862.1
(22) Anmeldetag: 04.08.2022
(51) Int. Cl.: C12M 1/00, A61M 39/18, C12M 1/34, G01D 11/30

(54) **BEFESTIGUNGSELEMENT ZUR ANORDNUNG AN EIN ANSCHLUSSELEMENT EINES PROZESSBEHÄLTERS**
FASTENING ELEMENT FOR MOUNTING ON A CONNECTING ELEMENT OF A PROCESS CONTAINER
ÉLÉMENT DE FIXATION DESTINÉ À ÊTRE AGENCÉ SUR UN ÉLÉMENT DE RACCORDEMENT D'UN RÉCIPIENT DE PROCESSUS

(43) Veröffentlichungstag der Anmeldung: 07.02.2024
(73) Patentinhaber: Exner & Tottewitz Besitz GBR, 76275 Ettlingen (DE)
(72) Erfinder: Tottewitz, Michael, 76646 Bruchsal (DE); Exner, Detlef, 71297 Mönsheim (DE); Hanselmann, Timo, 76199 Karlsruhe (DE); Güney, Yasar, 76275 Ettlingen (DE)
(74) Vertreter: LBP Lemcke, Brommer & Partner Patentanwälte mbB

(56) Entgegenhaltungen:
- WO-A1-2020/260089
- US-A1- 2011 124 035
- US-A1- 2013 145 818
- US-A1- 2014 260 712

## Beschreibung

Die Erfindung betrifft ein Befestigungselement zur Anordnung an ein Anschlusselement eines Prozessbehälters gemäß Anspruch 1. Weiterhin betrifft die Erfindung einen Prozessbehälter nach Anspruch 10.

Prozessbehälter werden beispielsweise in chemischen, biotechnischen und pharmazeutischen Anlagen verwendet. Innerhalb der Anlagen werden die Prozessbehälter verwendet, um in ihnen chemische, biologische oder pharmazeutische Prozesse ablaufen zu lassen. Der Prozessbehälter ist somit ein verfahrenstechnischer Apparat, wobei für die Durchführung der Verfahren Zustandsgrößen wie beispielsweise Temperatur, Druck oder auch Konzentration des Mediums innerhalb des Prozessbehälters geändert werden müssen.

Ein Beispiel eines Prozessbehälters aus dem biotechnologischen Bereich sind Bioreaktoren. Diese Bioreaktoren dienen meist der Kultivierung von Mikroorganismen zur Kultivierung von tierischen, menschlichen oder pflanzlichen Zellen.

Die Grundstoffe, welche durch die Bioreaktoren gewonnen werden, können zur Herstellung von Pharmazeutika genutzt werden. Bei Pharmazeutika ist eine hohe Reinheit des Produktes notwendig. Es ist somit bei der Herstellung von Pharmazeutika und deren Grundbausteine notwendig, eine hohe Prozesssicherheit zu gewähren, um einen möglichst geringen Ausschuss zu erzielen.

Um diese Prozesssicherheit zu gewähren, erfolgt typischerweise mittels einer Steuerungseinheit eine Steuerung oder Regelung des Prozesses.

Hierfür werden Informationen zum Zustand des Prozesses, die sogenannten Prozessparameter, benötigt. Die Prozessparameter werden typischerweise über eine Probenentnahme und einer Analyse der Proben bestimmt. Diese Entnahme von Proben birgt jedoch das Risiko einer Kontaminierung des sich im Prozessbehälter befindlichen Mediums, was zu einer fehlerhaften Produktionscharge führen kann. Damit nicht unmittelbar eine Probe des Mediums innerhalb des Prozessbehälters entnommen werden muss, um den Zustand des Prozesses zu erfassen, ist es bekannt, in Prozessbehälter, insbesondere in Bioreaktoren Anschlusselemente zu integrieren.

Diese Anschlusselemente dienen dazu, von außerhalb des Behälters Messungen im Inneren des Behälters durchzuführen. Hierfür wird ein Befestigungselement an das Anschlusselement des Behälters angelegt. Über das angelegte Befestigungselement, welches ein Messfenster aufweist, kann ein Sensor angeordnet werden, sodass der Sensor durch das Messfenster hindurch Messtrahlung wie beispielsweise Infrarotstrahlen aussendet, um beispielsweise eine Trübung des Mediums zu messen.

Ein solches Befestigungselement ist aus DE102019115147B4 bekannt: Über eine Schraubverbindung wird das Befestigungselement mit dem Anschlusselement des Prozessbehälters verbunden. Hierfür ist das Befestigungselement in einen Stutzen und in ein Festlegeelement unterteilt. Damit einer Kontaminierung des Mediums vorgebeugt wird, ist das Befestigungselement mit dem Anschlusselement des Behälters fluiddicht über das Festlegeelement verbunden.

Aus US2013/145818 A1 ist eine Sensoreinheit bekannt, welche an einem Behälter befestigt werden kann. Die Sensoreinheit weist eine Flanscheinheit mit einem Sensorhaltebereich, in dem eine Messungseinheit mit mindestens einem Messelement platziert ist auf. Eine Klemmvorrichtung dient zur Befestigung der Messeinheit am Sensorhaltebereich.

Um die Produktionskosten der Prozessbehälter insbesondere die Reinigungskosten bei der Nutzung des Prozessbehälters zu verringern, werden sogenannte Einwegprozessbehälter verwendet, insbesondere bei Bioreaktoren sind Einwegbioreaktoren durchaus gängig. Diese Einwegreaktoren besitzen typischerweise Anschlusselemente, an denen Befestigungselemente angeschlossen werden. Bedingt durch die einmalige Verwendung von Einwegreaktoren ist ebenso eine Einwegverwendung eines Befestigungselements üblich. Bei der Einwegvariante des Befestigungselements, muss das Material, aus dem das Befestigungselement gefertigt ist, nicht zwingend autoklavierbar sein. Somit können Kosten, insbesondere Materialkosten, bei der Fertigung des Befestigungselements eingespart werden.

Der vorliegenden Erfindung liegt die Aufgabe zugrunde, ein Befestigungselement der erwähnten Art so weiterzubilden, dass ein erneutes Lösen des Befestigungselements nach dem Festlegen an ein Anschlusselement eines Prozessbehälters verhindert wird.

Ein Risiko bei der Verwendung von Einwegbefestigungselemente an Einwegbioreaktoren besteht darin, dass eine potenzielle Wiederverwendung eines Befestigungselements, welches nur zur einmaligen Nutzung ausgelegt ist, an einem weiteren Behälter zu einer Kontaminierung des Mediums innerhalb des weiteren Behälters führen kann.

Darüber hinaus können bei Entfernen des Befestigungselementes Verunreinigungen in den Prozessbehälter gelangen und Medium kann aus dem Behälter austreten.

Das Risiko der erneuten Verwendung des Befestigungselements wird erfindungsgemäß über ein zusätzliches Sicherungselement vermieden. Über das Sicherungselement wird das festgelegte Festlegungselement gesichert.

Erfindungsgemäß weist das Befestigungselement zur Anordnung an ein Anschlusselement eines Prozessbehälters, insbesondere eines Reaktors, bevorzugt eines Bioreaktors, besonders bevorzugt eines Einwegbioreaktors, einen Stutzen und ein Festlegeelement auf. Der Stutzen weist hierbei eine Sensoröffnung zum Einführen eines Sensors in den Stutzen sowie ein an einer der Sensoröffnung gegenüberliegenden Seite ausgebildetes Messfenster auf. Das Festlegeelement ist ausgebildet, um den Stutzen an das Anschlusselement des Prozessbehälters anzuordnen.

Wesentlich ist hierbei, dass das Festlegeelement zumindest einen Rastarm mit einem beweglichen Rastbereich aufweist, um eine Rastverbindung mit dem Anschlusselement des Prozessbehälters auszubilden. Zudem weist das Festlegeelement zumindest ein verschiebbar an dem Festlegeelement angeordnetes Sicherungselement auf, wobei das Sicherungselement und das Festlegeelement derart zusammenwirkend ausgebildet sind, dass in einer Einführungsposition des Sicherungselementes der Rastbereich des zumindest einen Rastarms beweglich ist und in einer Sicherungsposition des Sicherungselementes eine Bewegung des Rastbereichs des zumindest einen Rastarms durch das Sicherungselement verhindert wird.

Vorteilhaft ist, dass durch den beweglichen Rastarm die Rastverbindung in einer einfachen Weise ausgeführt werden kann, sodass das Festlegeelement einfach über das Anschlusselement des Prozessbehälters geschoben werden kann und dadurch, dass der Rastarm beweglich ausgeführt ist, ein leichtes Zurückbiegen des Rastamtes dazu führt, dass der Rastbereich die eigentliche Rastverbindung mit dem Anschlusselement ausbildet. Vorteilhaft ist zudem, dass das Sicherungselement in der Einführungsposition den Rastarm nicht in seiner Beweglichkeit hindert.

Weiterhin ist vorteilhaft, dass durch eine einfache Bewegung des Sicherungselementes von der Einführungsposition hin zur Sicherungsposition eine Verringerung der Beweglichkeit des Rastbereiches des Rastarmes erzeugt wird.

Weiterhin ist vorteilhaft, dass für die Sicherung des Festlegungselementes des Befestigungselementes an dem Anschlusselement kein Sicherungsmittel wie Schraubensicherungskleber oder Verplombungen verwendet werden müssen.

Bei einer vorteilhaften Ausführungsform des Befestigungselements weist das Festlegeelement einen zylindrischen Grundkörper auf, der durch bevorzugt axial verlaufende Schlitze in mindestens zwei Rastarme unterteilt ist. Vorteilhaft ist bei dieser Ausführungsform, dass durch den Schlitz in einer einfachen Weise die Beweglichkeit der Rastbereiche der Rastarme gegeben wird, somit kann beim Positionieren des Festlegeelements an dem Anschlusselement des Prozessbehälters eine vom Zentrum her nach außen gerichtete Bewegung der Rastarme stattfinden, sodass ein Bereich des Anschlusselements durch das Festlegeelement hintergriffen wird. Besonders vorteilhaft ist es, wenn mehrere Schlitze dazu führen, dass eine Vielzahl von Rastarmen ausgebildet werden.

Bedingt durch den größeren Bewegungsfreiraum der Rastarme ist eine Positionierung des Festlegeelements an das Anschlusselement umso einfacher, je mehr Rastarme ausgebildet sind.

Bei einer weiteren vorteilhaften Ausführungsform ist das Sicherungselement ringförmig ausgebildet und umschließt das Festlegeelement zumindest im Rastbereich in der Sicherungsposition. Das ringförmige Sicherungselement verhindert, dass sich die beweglichen Rastarme und deren Rastbereiche des Festlegeelements bewegen können. Vorteilhaft ist dabei, dass nur ein Sicherungselement genutzt wird, um alle Befestigungsarme zu sichern.

Bei einer weiteren bevorzugten Ausführungsform ist das Sicherungselement ringförmig ausgebildet und umschließt das Festlegeelement in einer Einführungsposition. Vorteilhaft bei dieser Ausführungsform ist, dass somit das Sicherungselement schon an der Einführungsposition das Festlegeelement umschließt, sodass das Sicherungselement nicht zusätzlich positioniert werden muss.

Besonders vorteilhaft ist es, wenn das Sicherungselement in der Einführungsposition das Festlegeelement umschließt und das Befestigungselement einen Anschlag für das Sicherungselement aufweist, um ein Verschieben des Sicherungselementes aus der Einführungsposition entgegen der Richtung des Rastbereiches des Festlegeelements zu verhindern. Vorteilhafterweise ist der Anschlag für das Sicherungselement daher derart angeordnet und ausgebildet, dass das Sicherungselement in der Einführungsposition an der der Sicherungsposition abgewandten Seite an dem Anschlag anliegt.

Vorteilhaft ist bei dieser Ausführungsform, dass sich das Festlegeelement und das Sicherungselement vor dem Festlegen an dem Anschlusselement des Prozessbehälters aneinander anordnen lassen und dennoch ein Verlust des Sicherungselements vermieden wird, was insbesondere den Montageprozess erleichtert.

Bei einer weiteren vorteilhaften Ausführungsform des Befestigungselements ist das Sicherungselement und das Festlegeelement derart zusammenwirkend ausgebildet, dass das Sicherungselement und das Festlegeelement in der Sicherungsposition eine formschlüssig ineinandergreifende Rastverbindung ausbilden. Vorteilhaft bei dieser Ausführungsform ist, dass durch die formschlüssige Rastverbindung eine einfache und schnelle Montage des Befestigungselements an dem Anschlusselement des Prozessbehälters erfolgt. Ein weiterer Vorteil der Rastverbindung ist, dass die Rastung in einer einfachen Weise zusammengeführt werden kann, um eine Verbindung herzustellen, jedoch ist das Lösen der Rastverbindung durch die Geometrie der Rastelemente (insbesondere Rastnasen) erschwert. Weiterhin ist vorteilhaft, dass ein erneutes Lösen der Rastverbindung nur über die Zerstörung des Sicherungselements vollzogen werden kann. Es ist daher vorteilhaft, dass an dem Festlegeelement zumindest eine Rastnase ausgebildet ist.

Bei einer weiteren vorteilhaften Ausführungsform ist das Messfenster hermetisch dicht an dem Stutzen angeordnet, insbesondere über eine im Inneren des Stutzens liegende Fassung, bevorzugt einer stufenförmigen Fassung.

Vorteilhaft ist eine hermetisch dichte Anordnung des Messfensters an den Stutzen bedingt durch die benötigte Kontaminationsfreiheit des Mediums des Prozessbehälters. Weiterhin ist es von Vorteil, dass insbesondere durch die stufenförmige Ausbildung der Fassung bei Einführen eines Sensors in den Stutzen ein Herausdrücken des Messfensters in das Innere des Prozessbehälters verhindert wird.

Zudem ist weiter vorteilhaft, dass wenn die Fassung stufenförmig ausgebildet ist, dass die entstehenden Kräfte, die durch das Einführen des Sensors in den Stutzen entstehen, gleichmäßig in die Flächenbereiche der einzelnen Stufen eingebracht werden. Somit kann eine zu starke Belastung des Messfensters unterbunden werden.

In einer weiteren vorteilhaften Ausführungsform ist das Messfenster fluiddicht an dem Stutzen angeordnet.

Die fluide Dichtigkeit ist Voraussetzung, damit ein fluides Medium innerhalb des Prozessbehälters bleibt und nicht über das Befestigungselement aus dem Prozessbehälter gelangt.

In einer weiteren vorteilhaften Ausführungsform des Befestigungselements ist das Material des Stutzens biokompatibel, insbesondere aus Polyethylen (PE), bevorzugt aus Polyaryletherketon (PAEK), bevorzugt aus Polyethylenterephthalat (PET) oder aus Polypropylen (PP) oder aus Polyamid (PA) ausgebildet, insbesondere aus Polyetheretherketon (PEEK), bevorzugt aus Polyphenylensulfon (PPSU).

Hierbei ist es wichtig, dass es zu keiner Zersetzung des Materials des Stutzens kommt, sodass Abbauprodukte des Zersetzungsprozesses in das Medium gelangen und dort das Mediums verunreinigen. Zudem ist es vorteilhaft, Kunststoffe zu verwenden. Die Herstellungskosten sind geringer, verglichen mit auf metallischem Material basierenden Stutzen. Zudem lassen sich einfache Kunststoffteilfertigungsverfahren nutzen.

Bei einer vorteilhaften Ausführungsform des Befestigungselements ist in einem Bereich der Sensoröffnung des Stutzens eine Kupplung, bevorzugt ein Kupplungsstecker, insbesondere bevorzugt ein Schnellverschlusskupplungsstecker zur Anordnung eines Sensors ausgebildet. Vorteilhaft ist hierbei, dass durch die angeordnete Kupplung in einfacher Weise der Sensor an dem Befestigungselement angeordnet werden kann. Insbesondere bietet die Ausführungsform mit Schnellverschlusskupplung den Vorteil, auch ein schnelles Anbringen bzw. schnelles Lösen des Sensors insbesondere, wenn mit dem Sensor in einem kurzen Zeitraum mehrere Messungen in verschiedenen Prozessbehälter oder in verschiedenen Befestigungselementen desselben Prozessbehälters durchgeführt werden.

In einer weiteren vorteilhaften Ausführungsform ist das Material des Messfensters transparent, bevorzugt aus Glas, insbesondere aus Glaskeramik, besonders bevorzugt aus Saphirglas ausgebildet.

Vorteilhaft ist bei dieser Ausführungsform, dass durch ein transparentes Messfenster Messstrahlen, welche von einem Sensor in Richtung des Messfensters emittiert werden, durch das Material des Messfensters nicht oder nur geringfügig absorbiert werden, gleiches gilt für einkommende, im Medium reflektierte oder erzeugte Strahlung. Daher weist das Messfenster bevorzugt eine Transparenz in einem Wellenlängenbereich, der dem Messbereich des Sensors entspricht, bevorzugt in einem Wellenlängenbereich von 100 nm bis 2000 nm auf. Der besondere Vorteil bei der Verwendung von Saphirglas oder Glaskeramik ist, die besonders kratzfeste Oberfläche der Gläser, sowie die mit geringer Dämpfung beaufschlagte Transmissionsmöglichkeit der elektromagnetischen Wellen durch die Materialien.

Bei einer weiteren vorteilhaften Ausführungsform des Befestigungselements weist das Festlegeelement mehrere, sich nach außen öffnende Rastarme auf. Die Rastarme bilden bevorzugt einen an der Seite mit dem größeren Durchmesser offenen Kegelstumpf aus.

Vorteilhaft bei dieser Ausführungsform ist, dass der Abstand zwischen Festlegeelement und Anschlusselement somit im Bereich der Rastarme größer ist wie bei der Ausführungsform mit nicht nach außen geöffneten Rastarmen Dieser größere Abstand führt zu einem einfachen Verschieben des Sicherungselements aus der Einführungsposition zu der Sicherungsposition. Vorteilhaft ist zudem, dass durch die sich nach außen öffnenden Rastarme beim Positionieren des Sicherungselements in der Sicherungsposition bedingt durch die Federwirkung der Rastarme eine Federkraft auf das Sicherungselement wirkt. Die Federkraft unterstützt das Halten der Sicherungsposition und erschwert ein Lösen des Sicherungselements in der Sicherungsposition.

Bei einer weiteren vorteilhaften Ausführungsform des Befestigungselements ist das Sicherungselement in der Einführungsposition lösbar feststellbar. Vorteilhaft ist hierbei, dass das Sicherungselement daran gehindert wird ungewollt in die Sicherungsposition zu gelangen.

Bei einer weiteren vorteilhaften Ausführungsform des Befestigungselements ist das Sicherungselement ringförmig ausgeführt und weist ein Innengewinde auf und das Festlegeelement weist einen Bereich mit einem Außengewinde auf. Das Sicherungselement ist zunächst an der Einführungsposition angeordnet, sodass der Bereich mit dem Außengewinde des Führungselements und das Innengewinde des Sicherungselements ineinandergreifen. Das Sicherungselement wird durch eine Drehbewegung aus der Einführungsposition hin zu der Sicherungsposition geführt. Beim Erreichen der Sicherungsposition des Sicherungselements wird eine Bewegung des Rastarms durch das Sicherungselement verhindert. Vorteilhaft ist bei dieser Ausführungsform, dass der rückstellenden Kraft welche bei einem Festlegeelement mit gebogenen Rastarmen auftritt welche dazu führt, dass das Sicherungselement entgegen der Richtung der Sicherungsposition hin zu der Einführungsposition gedrückt wird, durch die der Rückstellkraft entgegen gerichtete Schraubenlängskraft entgegengewirkt wird. Vorteilhaft ist hierbei das ein Einfaches Festlegen des Befestigungselements an dem Festlegeelement möglich ist.

Bei dem erfindungsgemäßen Prozessbehälter, insbesondere zur Züchtung von Zellkulturen oder Mikroorganismen in einem Medium mit zumindest einem Anschlusselement mit angeordnetem erfindungsgemäßen Befestigungselement ist wesentlich, dass das Anschlusselement des Prozessbehälters durch die Anordnung des Befestigungselements an das Anschlusselement fluiddicht ist, insbesondere derart, dass das Messfenster an einen Innenraum des Prozessbehälters angrenzt.

Vorteilhaft ist bei diesem erfindungsgemäßen Prozessbehälter, dass durch die fluiddichte Verbindung des Befestigungselements mit dem Anschlusselement des Prozessbehälters keine fluiden Medien aus dem Prozessbehälter über das Befestigungselement an die Umwelt gelangen kann. Zudem ist es vorteilhaft, dass das Messfenster auch in das Innere des Prozessbehälters gerichtet ist um Messungen im Inneren des Messbehälters durchzuführen.

Bei einer vorteilhaften Ausführungsform des Prozessbehälters ist das Anschlusselement durch einen Flansch mit einem angeformten Adapter ausgebildet, bevorzugt einem angeformten Schlauchadapter, insbesondere bevorzugt mit einem angeformten rohrförmigen Adapter, besonders bevorzugt mit einem angeformten rohrförmigen Adapter, welcher eine kreiszylindrische Innenseite aufweist und außen einen kegelstumpfförmigen Bereich aufweist, insbesondere besonders bevorzugt mit einem angeformten rohrförmigen Adapter, welcher eine kreiszylindrische Innenseite aufweist und außen mehrere axiale Bereiche mit sich unterscheidenden Außendurchmesser aufweist.

Vorteilhaft ist bei dieser Ausführungsform, dass das Festlegeelement des Befestigungselements beispielsweise den kegelstumpfförmigen Bereich umschließt, sodass die Rastarme an den Bodenbereich des kegelstumpfförmigen Bereiches einrasten können und bedingt durch die Kegelstumpfform ein Ausweichbereich ausgebildet wird, in welchen der bewegbare Rastbereich des zumindest einen Rastarmes bis zum Erreichen der Sicherungsposition des Sicherungselements ausweichen kann. Zudem ist es vorteilhaft, die Innenseite des Anschlusselements kreiszylindrisch auszugestalten, sodass der Stutzen in einer einfachen Art und Weise durch das Anschlusselement geführt werden kann und eine einfache Passung mit einem Stutzen mit kreiszylindrischer Außenseite erzielt werden kann.

Der Stutzen des Befestigungselements weist daher bevorzugt eine kreiszylindrische Außenseite auf, insbesondere ist der Stutzen bevorzugt kreiszylindrisch ausgebildet.

Vorteilhaft ist, dass durch den Flansch des Anschlusselements in einer einfachen Art und Weise eine Befestigung an den Prozessbehälter umgesetzt werden kann. Da jedoch ein Flansch nur eine Öffnung ausbildet, ist es wesentlich, dass an dem Flansch auch ein Adapter ausgebildet ist. Dieser Adapter dient zum Positionieren des Befestigungselementes.

In einer weiteren vorteilhaften Ausführungsform des Prozessbehälters ist das Material des Anschlusselements biokompatibel, insbesondere aus Polyethylen (PE), bevorzugt aus Polyaryletherketon (PAEK), bevorzugt aus Polyethylenterephthalat (PET), oder aus Polypropylen (PP) oder aus Polyamid (PA), insbesondere aus Polyetheretherketon (PEEK), bevorzugt aus Polyphenylensulfon (PPSU).

Vorteilhaft ist bei der Ausführung des Anschlusselements als biokompatibles Anschlusselement, dass bei Kontakt des Anschlusselements mit dem Fluid des Prozessbehälters keine Zersetzungsprodukte entstehen, welche in das Medium des Prozessbehälters übergehen. Ein weiterer Vorteil der Verwendung von Materialien aus der Gruppe der Kunststoffe ist, dass diese in einer einfachen Art und Weise über bekannte Herstellungsverfahren kostengünstig hergestellt werden können.

Bei einer weiteren vorteilhaften Ausführungsform des Prozessbehälters sind das Festlegeelement und das zumindest eine Anschlusselement des Prozessbehälters konzentrisch angeordnet, insbesondere sind bevorzugt das Festlegeelement, das Sicherungselement und das Anschlusselement konzentrisch angeordnet. Hierdurch ist ein einfaches Anordnen der einzelnen Elemente möglich, sodass zunächst der Stutzen in das Anschlusselement eingeführt wird und darauffolgend das Festlegeelement konzentrisch an dem Anschlusselement angeordnet wird, sodass der Stutzen über das Festlegeelement an dem Anschlusselement angeordnet ist und das Sicherungselement in einer einfachen Art und Weise konzentrisch zum Festlegeelement im Anschlusselement angeordnet ist. Ein weiterer Vorteil für die konzentrische Anordnung der Bauelemente ist der geringe Platzaufwand.

Bei einer weiteren vorteilhaften Ausführungsform des Prozessbehälters ist zwischen dem Festlegeelement und dem Anschlusselement ein Ausweichbereich ausgebildet, in welchen der bewegbare Rastbereich des zumindest einen Rastarmes bis zum Erreichen der Sicherungsposition des Sicherungselementes ausweichen kann, insbesondere durch Biegen des Rastarmes.

Vorteilhaft ist bei dieser Ausführungsform, dass über das Ausbilden des Ausweichbereiches in einer einfachen Art und Weise die Sicherungsfunktion des Sicherungselementes umgesetzt werden kann. Denn durch die Ausweichbewegung der Rastarme in den Ausweichbereich ist es möglich, dass ringförmig ausgebildete Sicherungselement in einer einfachen Art und Weise in die Sicherungsposition zu bringen. Beim Erreichen der Sicherungsposition wird die Biegung der Rastarme und somit auch das Ausweichen in den Ausweichbereich zurückgenommen.

In einer weiteren vorteilhaften Ausführungsform ist in dem Stutzen des Befestigungselements des Prozessbehälters ein Sensor angeordnet. Der Sensor ist als optoelektronischer Sensor, insbesondere als Trübheitssensor ausgebildet. Der Vorteil bei der Befestigung des Sensors an das Befestigungselement ist, dass somit der Sensor geführt bzw. gefasst vom Befestigungselement eine Messung in das Innere des Behälters durch das Messfenster ermöglicht. Weiterhin ist vorteilhaft, dass durch einen optoelektronischen Sensor eine möglichst störungsfreie Messung innerhalb des Mediums durchgeführt wird. Durch das Messen mit dem Sensor über das Befestigungselement müssen keine Proben entnommen werden, um eine Messung im Medium durchzuführen.

Bei einer vorteilhaften Weiterbildung der zuvor genannten Ausführungsform des Prozessbehälters mit Sensor ist in einem Bereich der Sensoröffnung des Stutzens des Befestigungselements eine Kupplung bevorzugt ein Kupplungsstecker, insbesondere ein Schnellverschlusskupplungsstecker zur Anordnung des Sensors ausgebildet. Insbesondere ist es vorteilhaft, dass der Sensor mittels der Kupplung des Stutzens an dem Befestigungselement angeordnet ist, insbesondere über eine Kupplungsdose des Sensors, bevorzugt eine Schnellverschlusskupplungsdose des Sensors.

Vorteilhaft ist bei dieser Ausführungsform, dass der Sensor in einer einfachen Art und Weise an dem Befestigungselement positioniert werden kann. Vorteilhaft ist zudem, dass insbesondere bei der Verwendung der Schnellverschlusskupplung eine Einhandpositionierung des Sensors innerhalb des Befestigungselements möglich ist.

Weitere vorteilhafte Merkmale und Ausgestaltungen werden nachfolgend anhand von Ausführungsbeispielen und den Figuren 1 bis 4 gezeigt. Im Folgenden zeigt:
- Figur 1:: Teilabbildungen verschiedener Ausführungsbeispiele von Befestigungselementen an einem Anschlusselement eines Prozessbehälters;
- Figur 2:: ein weiteres Ausführungsbeispiel eines Befestigungselementes mit einem Stutzen, Sicherungselement sowie Anschlusselement gemäß Figur 1a
- Figur 3:: Ausführungsbeispiele von Festlegeelementen des Befestigungselements in einer Ansicht von unten, sowie ein Ausführungsbeispiel mit nach außen verlaufenden Rastarmen;
- Figur 4:: Ausführungsbeispiel eines im Schnitt gezeigten Befestigungselements an einem Anschlusselement eines Prozessbehälters mit und ohne angebrachten Sensor.

Sämtliche Figuren zeigen schematische, nicht maßstabsgetreue Darstellungen. Gleiche Bezugszeichen in den Figuren bezeichnen gleiche oder gleichwirkende Elemente.

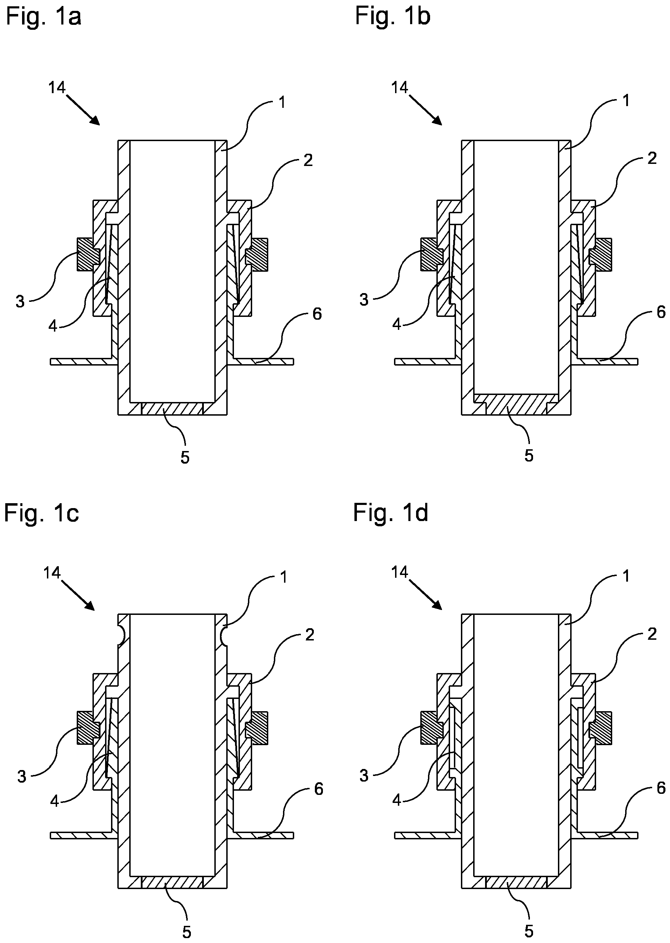

Die Figuren zeigen jeweils Ausführungsbeispiele erfindungsgemäßer Befestigungselemente. In den Figuren 1, 2 und 4 sind weiterhin Anschlusselemente von Ausführungsbeispielen erfindungsgemäßer Prozessbehälter gezeigt.

Das in Teilabbildung 1a gezeigte Ausführungsbeispiel eines Befestigungselements 14 zeigt die einzelnen Elemente des Befestigungselements 14: den Stutzen 1, das Festlegeelement 2 und das Sicherungselement 3. Gezeigt ist der festgelegte Zustand des Befestigungselements 14 am Anschlusselement 6 eines Prozessbehälters. Hierbei ist das Anschlusselement 6 des Prozessbehälters als Flansch ausgeführt mit angeformten Adapter 4. Dieser angeformte Adapter 4 ist rohrförmig ausgebildet und ist innen zylinderförmig und weist außen einen Bereich auf, der kegelstumpfförmig ausgebildet ist. Alle Elemente sind im Querschnitt dargestellt. Der Stutzen 1 des Befestigungselements 14 weist in einem unteren Bereich ein Messfenster 5 auf. Das Messfenster 5 ist in diesem Ausführungsbeispiel aus Glas gefertigt. Der Stutzen 1 des Befestigungselements 14 ist in diesem Beispiel gefertigt aus PEEK. Bei diesem Ausführungsbeispiel ist der Grundkörper des Stutzens 1, der Grundkörper des Festlegeelements 2 sowie des Sicherungselementes 3 und das Anschlusselementes rotationssymmetrisch ausgebildet. Der Stutzen 1 ist rohrförmig ausgeführt und weist eine Sensoröffnung zum Einführen eines Sensors in den Stutzen 1 auf. Diese Sensoröffnung befindet sich gemäß Darstellung in Figur 1 am oberen Ende des Stutzens 1. Weiterhin ist am Stutzen 1 entlang des Umfangs in einem oberen Bereich des Stutzens 1 ein Steg ausgeformt. Dieser Steg ist ringförmig ausgeführt und dient zum Anlegen des Stutzens 1 an die Oberseite des kegelstumpfförmigen Bereiches 4 des Anschlusselementes des Prozessbehälters über das Festlegeelement 2 des Befestigungselements. Die Länge des rohrförmigen Adapters 4 ist kürzer als die des Stutzens 1, sodass sich der Stutzen 1 teilweise in den Innenraum des Prozessbehälters erstreckt.

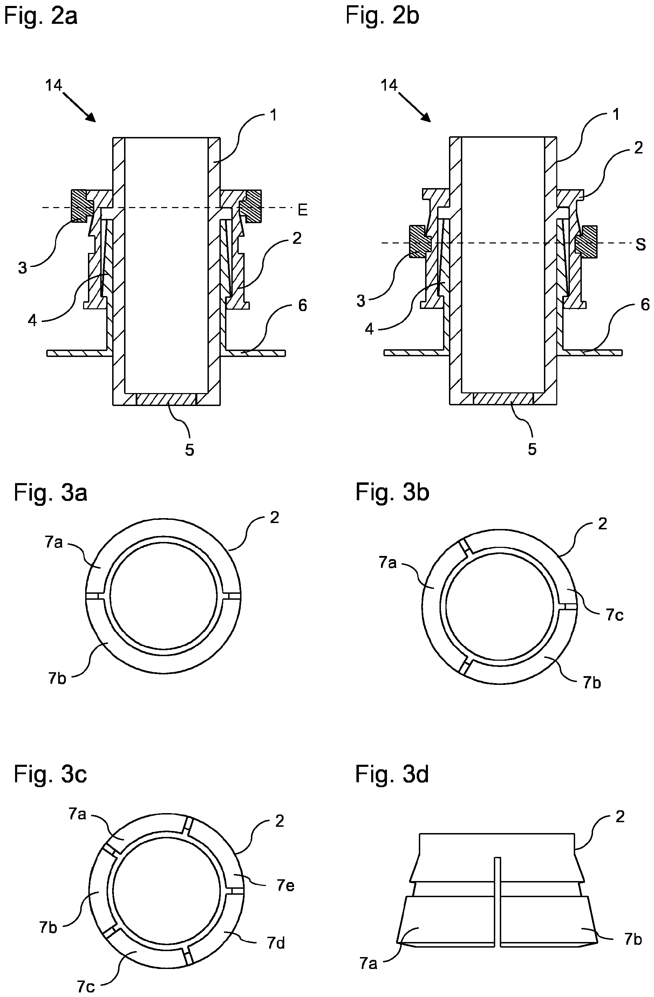

Zum Festlegen des Stutzens 1 an das Anschlusselement 6 des Prozessbehälters weist das Festlegeelement 2 durch zwei Schlitze getrennte Rastarme auf, gemäß der Ausgestaltung der Figur 3a. Das Sicherungselement 3 ist verschiebbar an dem Festlegelement 2 angeordnet und wird von der gemäß Darstellung in Figur 1a oberhalb der Sicherungsposition, liegenden Einführungsposition, in Richtung der Sicherungsposition nach unten verschoben, sodass das Sicherungselement 3 bei dem Verschiebungsprozess die Rastarme nach innen in Richtung eines Ausweichbereiches des Anschlusselements 6 drückt bis zum Erreichen der Sicherungsposition am Festlegeelement 2. Der zuvor genannte Ausweichbereich stellt den Bereich dar, welcher zwischen der Außenfläche des Anschlusselements in diesem Fall die Außenfläche des kegelstumpfförmigen Grundkörpers des Anschlusselements 6 und der Innenfläche der Rastarme des Festlegungselements 2 entsteht. In diesem Beispiel ist der Ausweichbereich bedingt durch die kegelstumpfförmige Ausgestaltung des oberen Bereiches des angeformten Bereiches im oberen Bereich größer als der hin zum Boden des kegelstumpfförmigen Bereiches verlaufende Ausweichbereich. Nach dem Erreichen der Sicherungsposition rastet das Sicherungselement 3 mit einem innenliegenden, umlaufenden Ring in den als außenliegende, umlaufende Nut ausgebildeten Rastbereich des Festlegeelementes 2 ein. Das Sicherungselement 3 wird durch die Verrastung daran gehindert, die Sicherungsposition 3 zu verlassen. Durch das Sicherungselement wird eine Bewegung der Rastarme nach außen und somit ein Lösen des Befestigungselements von dem Anschlusselement 6 verhindert.

Bei diesem Ausführungsbeispiel weist der Stutzen 1 des Befestigungselementes keine ausgeformte Kupplungsmöglichkeit auf. Jedoch ist es möglich, einen Sensor in das rohrförmige Innere des Stutzens 1 einzuführen. Hierbei wird der Sensor an der Innenwandung des Stutzens 1 über eine Haltelippe des Sensors gehalten. Das in diesem Fall aus Glas bestehende Messfenster ist in diesem Ausführungsbeispiel in einer Öffnung des Stutzenbodens verklebt, welcher der Sensoröffnung gegenüberliegt.

Das in Figur 1b gezeigte Ausführungsbeispiel unterscheidet sich von dem Ausführungsbeispiel, welches in Figur 1a gezeigt ist durch die Befestigung des Glases des Messfensters 5 in dem Stutzenboden des Befestigungselements. Hierbei ist die Aufnahme des Glases stufenförmig gestaltet, sodass das Glas sich durch den Boden des Stutzens 1 in den Innenraum des Stutzens 1 erstreckt und innerhalb des Stutzenbodens einen kleineren Durchmesser aufweist wie im Bereich des inneren des Stutzens 1. Zum Festlegen des Stutzens 1 an das Anschlusselement 6 des Prozessbehälters ist das Festlegeelement 2 gemäß der Ausgestaltung der Figur 3a ausgebildet und weist zwei durch Schlitze getrennte Rastarme auf.

Die stufenförmige Ausführung der Fassung des Glases hat zum Vorteil, dass beim Einführen des Sensors in die Öffnung des Stutzens ein Herausdrücken des Glases des Messfensters 5 verhindert wird. Zudem kann über die größere Fläche, welche durch die Positionierung einer Stufenform zwischen dem Stutzen und dem Glas entsteht, eine bessere Verklebung bzw. Abdichtung erzielt werden. Weiterhin ist ein Vorteil des Ausführungsbeispiels, dass bei Prozessen im Prozessbehälter, welche bei niedrigerem Druck wie dem Außendruck der Umgebungsluft durchgeführt werden, das Glas des Messfensters 5 an den Boden des Stutzens 1 gedrückt wird. Somit kann die entstehende Kraft an der stufenförmigen Fassung besser übertragen werden.

Das Ausführungsbeispiel, welches in Figur 1c gezeigt ist, unterscheidet sich zu dem in Figur 1a gezeigten Ausführungsbeispiel durch die Möglichkeit der Kupplung eines Sensors in dem Bereich der Sensoröffnung des Stutzens 1. In diesem Ausführungsbeispiel ist in dem Bereich der Sensoröffnung des Stutzens 1 ein Kupplungsstecker ausgebildet. Dieser Kupplungsstecker weist eine in Umfangrichtung verlaufende Ausnehmung auf. Diese Ausnehmung ermöglicht es, eine Dose an den Stecker anzulegen, welche in diese Ausnehmung greift, um eine Verbindung zwischen dem Befestigungselement 14 und dem an der Dose befestigten Sensor zu ermöglichen. Zum Festlegen des Stutzens 1 an das Anschlusselement 6 des Prozessbehälters ist das Festlegeelement 2 gemäß der Ausgestaltung der Figur 3c ausgebildet und weist fünf durch Schlitze getrennte Rastarme auf.

Das in Figur 1d gezeigte Ausführungsbeispiel unterscheidet sich zu dem in Figur 1a gezeigten Ausführungsbeispiel durch die Ausgestaltung des Anschlusselements des Prozessbehälters. Im Gegensatz zu der kegelstumpfförmigen Ausgestaltung des oberen Bereiches 4 des rohrförmigen Stückes des als Flansch ausgeführten Anschlusselements 6, weist das Ausführungsbeispiel in Figur 1d einen zylinderförmigen Bereich auf, welcher eine umfangsseitige nutförmige Ausnehmung aufweist. Diese nutförmige Ausnehmung ermöglichte es, einen gleichbleibenden Ausweichbereich innerhalb der Nut.

Das in Figur 2a gezeigte Ausführungsbeispiel eines Befestigungselements unterscheidet sich zu den in Figur 1 gezeigten Ausführungsbeispielen des Befestigungselements durch die Ausgestaltung des Festlegeelements 2. In Figur 2a ist das Sicherungselement 3 in der Einführungsposition E dargestellt. Bei dieser Ausführungsform ist das Sicherungselement 3, welches ringförmig ausgebildet ist, beweglich an dem Festlegeelement 2 angeordnet. Das Sicherungselement 3 wird jedoch durch eine Ausbuchtung des Festlegeelements 2 daran gehindert, von dem Festlegeelement 2 getrennt zu werden. Die Ausbuchtung bildet somit einen Anschlag für das Sicherungselement 3 aus, um ein Verschieben des Sicherungselements 3 gemäß Figur 2 nach oben zu verhindern.

Das Festlegeelement 2 bildet eine Lagerung des Sicherungselements 3 in der Sicherungsposition S aus. Weiter wird das Sicherungselement 3 in der Einführungsposition E daran gehindert, selbstständig die Sicherungsposition S zu gelangen, da das Festlegelement 2 einen kegelstumpfförmigen Bereich aufweist, der eine selbstständige Bewegung des Sicherungselements 3 in Richtung der Sicherungsposition 3 hemmt.

Das Festlegeelement 2 weist in diesem Ausführungsbeispiel fünf durch Schlitze getrennte Rasterarme auf. In Figur 2a ist das Festlegeelement 2 an dem Anschlusselement 6 des Prozessbehälters festgelegt, aber noch nicht in einem gesicherten Zustand. Um das Befestigungselement 14 über das Festlegeelement 2 und das ringförmige Sicherungselement 3 an dem Abschlusselement 6 des Prozessbehälters festzulegen und zu sichern, wird das Sicherungselement 3 in Richtung des Stutzenbodens verschoben. Die beweglichen Rastarme werden hierbei in den Ausweichbereich zwischen dem Festlegeelement 3 und dem Anschlusselement 6 gebogen. Durch das somit verdrängte Material des Festlegeelements 2 in dem kegelstumpfförmigen Bereich wird es dem Sicherungselement 3 ermöglicht, die Sicherungsposition S zu erreichen.

In Figur 2b ist das Sicherungselement 3 in der Sicherungsposition S des Befestigungselements dargestellt. Beim Erreichen der Sicherungsposition S federn die biegsamen Rastarme aus und werden durch den formschlüssigen Rastbereich zwischen dem Festlegeelement 2 und dem Sicherungselement 3 gesichert. Weiter wird durch die Rastarme entstehende Federkraft die formschlüssige Rastverbindung zum Sicherungselement 3 und dem Festlegeelement 2 verbessert. Das Festlegeelement 2 weist an der der Einführungsposition E gegenüberliegenden Seite eine weitere Ausbuchtung auf. Diese weitere Ausbuchtung kommt zum Tragen, wenn das Festlegeelement 2 noch nicht an dem Stutzen 1 bzw. dem Anschlusselement 6 des Prozessbehälters angelegt ist und soll das Sicherungselement 3 daran hindern, bedingt durch die biegsamen Rastarme von dem Festlegeelement 2 getrennt zu werden.

Die Teilabbildungen der Figur 3 die Figuren 3a bis 3c zeigen jeweils unterschiedliche Ausführungsbeispiele des Festlegeelements 2, so unterscheiden sich diese durch die Anordnung bzw. durch die Aufteilung und Anzahl der Rastarme 7 des Festlegeelements 2. Das in Figur 3a gezeigte Festlegeelement 2 weist eine kreisförmige Öffnung auf, welche an der Oberseite kleiner ist als an der Unterseite. Weiter weist das Festlegeelement 2 einen Bereich zwischen der Ober- und Unterseite auf, welche einen größeren Innendurchmesser aufweist als die Öffnungsgröße der Öffnungen an der Ober- und Unterseite. Unterteilt ist das Festlegeelement 2 durch zwei axial verlaufende Schlitze. Diese Schlitze verlaufen von der Unterseite des Festlegeelements 2 hinzu der Oberseite des Festlegungselementes 2, wobei die Schlitze nicht durchgängig, sondern beabstandet von der Oberseite des Festlegeelements 2 enden. Somit werden die Rastarme 7 des Festlegeelements 2 ausgebildet. In diesem Ausführungsbeispiel werden durch die zwei Schlitze zwei Rastarme 7a/7b ausgebildet.

In Teilabbildung 3b ist ein Festlegeelement 2 gezeigt, welches sich zu dem in Figur 3a gezeigten Festlegeelement 2 dahingehend unterscheidet, dass dieses Festlegeelement 2 drei Schlitze aufweist und somit drei ausgebildete Rastarme 7a/7b/7c aufweist.

In Teilabbildung 3c ist ein Ausführungsbeispiel eines Festlegeelements 2 gezeigt, welches über fünf Schlitze fünf Rastarme 7a-7e ausbildet.

In Teilabbildung 3d ist ein Ausführungsbeispiel eines Festlegeelements 2 gezeigt, welches über durch zwei Schlitze getrennte Rastarme 7a/7b aufweist. Bei dieser Ausführungsform weisen die Rastarme 7a/7b eine Biegung auf. Die gebogenen Rastarme erstrecken sich nach außen, sodass die Rastarme 7a/7b eine Spreizung aufweisen. Die Rastarme bilden somit einen geschlitzten, kegelstumpfartigen Körper aus, wobei die Seite des Kegelstumpfs mit dem größeren Radius dem Boden des Stutzens 1 zugewandt ist.

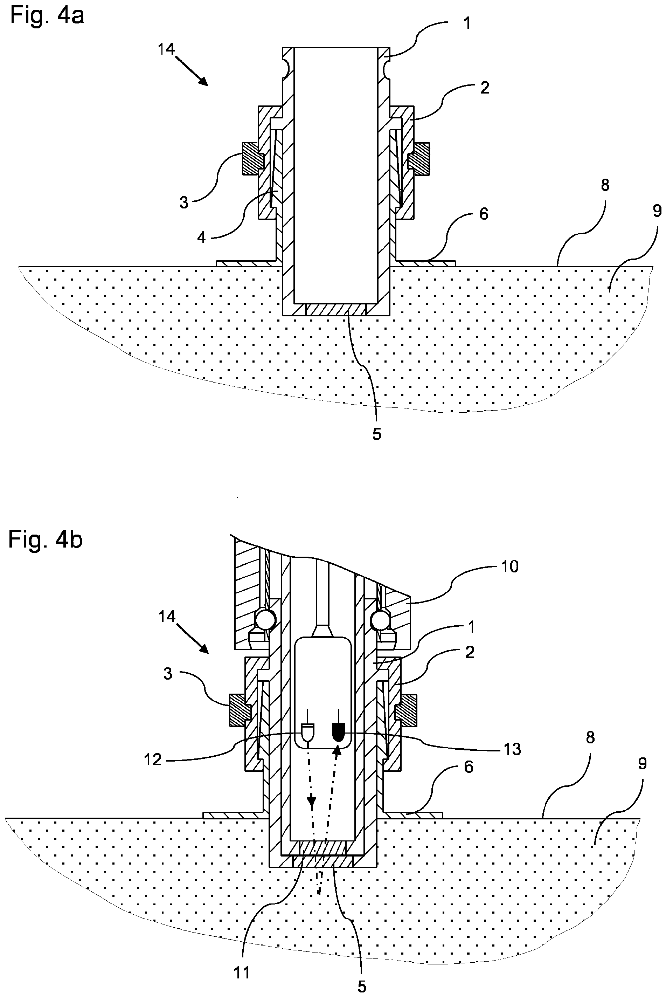

In Figur 4a ist ein Ausführungsbeispiel eines Befestigungselementes gezeigt, welches an einem Anschlusselement 6 eines Ausführungsbeispiels eines erfindungsgemäßen Prozessbehälters 8 angeordnet ist. In diesem Ausführungsbeispiel ist der Prozessbehälter 8 ein Einwegbioreaktor. In diesem Einwegbioreaktor werden in diesem Ausführungsbeispiel Zellkulturen für die Pharmazie hergestellt. Das Befestigungselement 14, insbesondere der Stutzen 1 des Befestigungselements 14 sowie das Messfenster 5 des Stutzens 1 ragen in den Einwegbioreaktor hinein. Der Bioreaktor 8 ist in diesem Falle mit einem Zellkulturmedium 9 gefüllt. Das Messfenster 5 steht im direkten Kontakt zu diesem Zellkulturmedium 9. Der Stutzen 1 weist in diesem Ausführungsbeispiel einen Kupplungsstecker auf. Das Befestigungselement 14 ist über das Sicherungselement 3 gesichert und über das Festlegeelement 2 an dem Anschlusselement 6 des Bioreaktors 8 festgelegt.

In Figur 4B ist das Befestigungselement 14 welches in Figur 4a gezeigt ist über eine Kupplungsdose, welche an einem Sensor 10 angeordnet ist, an diesen Sensor gekoppelt. In diesem Ausführungsbeispiel ist der Sensor 10 stabförmig ausgebildet und weist an der Unterseite ein Messfenster 11 auf. Der stabförmige Sensor 10 ist innerhalb des zylinderförmigen Innenbereiches des Stutzens 1 angeordnet, sodass das Messfenster 11 des Sensors und das Messfenster 5 des Stutzens 1 in Kontakt stehen. Die an dem Sensor 10 befestigte Kupplungsdose ist an den Kupplungsstecker des Festlegeelements 2, insbesondere des Kupplungssteckers des Stutzens 1 über einen Kugelverschluss gekuppelt.

Bei diesem Ausführungsbeispiel ist der Sensor 10 dazu ausgebildet, eine Trübheitsmessung in das sich in dem Einwegbioreaktor 8 befindlichen Zellkulturmediums 9 durchzuführen. Hierfür weist der Sensor 10 eine Lichtquelle 12 auf, welche im infraroten Wellenlängenbereich ein Signal aussendet (schematisch über einen Pfeil dargestellt). Dieses Signal durchläuft den Innenbereich des Sensors 10 und durchdringt das Messfenster 11 des Sensors sowie das Messfenster 5 des Stutzens 1, um in den Innenbereich des Bioreaktors 8 zu strahlen. Einen Teil des ausgesendeten inforoten Lichtsignals wird durch die sich in dem Zellkulturmedium 9 befindlichen Zellen reflektiert und wieder in Richtung des Messfensters 5 des Stutzens 1 zurückgeführt. Das zurückgeführte Signal gelangt durch das Messfenster 5 des Stutzens 1 sowie das Messfenster 11 des Sensors und durch einen Innenbereich des Sensors zu dem Empfänger 13 des Sensors 10. Über die Trübheit des sich in dem Bioreaktor befindlichen Zellkulturmediums 9 kann auf die Konzentration der Zellen in dem Medium 9 geschlossen werden. Durch die lösbare Verbindung der Kupplungsdose mit dem Kuppelstecker kann der Sensor nach der Messung einer einfachen Art und Weise von dem Befestigungselement 14 entfernt werden.

## Patentansprüche

1. Befestigungselement zur Anordnung an ein Anschlusselement (6) eines Prozessbehälters (8), insbesondere eines Reaktors, bevorzugt eines Bioreaktors, besonders bevorzugt eines Einwegbioreaktors,
wobei das Befestigungselement einen Stutzen (1) und ein Festlegeelement (2) aufweist,
wobei der Stutzen (1) eine Sensoröffnung zum Einführen eines Sensors in den Stutzen (1) und an einer der Sensoröffnung gegenüberliegenden Seite ein Messfenster (5) aufweist,
und wobei das Festlegeelement (2) ausgebildet ist, um den Stutzen (1) an dem Anschlusselement (6) des Prozessbehälters (8) anzuordnen,
**dadurch gekennzeichnet,**
**dass** das Festlegeelement (2) zumindest einen Rastarm (7) mit einem beweglichen Rastbereich aufweist, um eine Rastverbindung mit dem Anschlusselement (6) des Prozessbehälters (8) auszubilden
und das Festlegelement (2) zumindest ein verschiebbar an dem Festlegeelement (2) angeordnetes Sicherungselement (3) aufweist, wobei das Sicherungselement (3) und das Festlegeelement (2) derart zusammenwirkend ausgebildet sind, dass in einer Einführungsposition (E) des Sicherungselementes (3) der Rastbereich des zumindest einen Rastarms (7) beweglich ist und in einer Sicherungsposition (S) des Sicherungselementes (3) eine Bewegung des Rastbereichs des zumindest einen Rastarms (7) durch das Sicherungselement (3) verhindert wird.

2. Befestigungselement nach Anspruch 1,
**dadurch gekennzeichnet,**
**dass** das Festlegeelement (2) einen bevorzugt zylindrischen Grundkörper aufweist, der durch bevorzugt axial verlaufende Schlitze in mindestens zwei Rastarme (7) unterteilt ist.

3. Befestigungselement (14) nach Anspruch 1 und 2,
**dadurch gekennzeichnet,**
**dass** das Sicherungselement (3) ringförming ausgebildet ist und das Festlegeelement zumindest im Rastbereich in der Sicherungsposition umschließt.

4. Befestigungselement (14) nach Anspruch 1, Anspruch 2 und Anspruch 3,
**dadurch gekennzeichnet,**
**dass** das Sicherungselement (3) und das Festlegeelement (2) derart zusammenwirkend ausgebildet sind, dass das Sicherungselement (3) und das Festlegeelement (2) in der Sicherungsposition (S) eine formschlüssig ineinandergreifende Rastverbindung ausbilden.

5. Befestigungselement (14) nach Anspruch 1,
**dadurch gekennzeichnet,**
**dass** das Messfenster (5) hermetisch dicht an dem Stutzen (1) angeordnet ist, insbesondere über eine im Inneren des Stutzens (1) liegende Fassung, bevorzugt eine stufenförmige Fassung.

6. Befestigungselement (14) nach Anspruch 1,
**dadurch gekennzeichnet,**
**dass** das Material des Stutzens (1) biokompatibel ist, insbesondere aus Polyethylen (PE) ist, bevorzugt aus Polyaryletherketon (PAEK) ist, insbesondere bevorzugt aus Polyethylenterephthalat (PET) oder aus Polypropylen (PP), aus Polyamid (PA), insbesondere aus Polyetheretherketon (PEEK), bevorzugt aus Polyphenylensulfon (PPSU) ist.

7. Befestigungselement (14) nach Anspruch 1,
**dadurch gekennzeichnet,**
**dass** in einem Bereich der Sensoröffnung des Stutzens (1) eine Kupplung bevorzugt ein Kupplungsstecker, insbesondere ein Schnellverschlusskupplungsstecker zum Anordnen eines Sensors (10) ausgebildet ist.

8. Befestigungselement (14) nach Anspruch 1,
**dadurch gekennzeichnet,**
**dass** das Material des Messfensters (5) transparent ist, bevorzugt aus Glas ist, insbesondere aus Glaskeramik ist, besonders bevorzugt aus Saphirglas ist.

9. Befestigungselement (14) nach Anspruch 1,
**dadurch gekennzeichnet,**
**dass** das Sicherungselement (2) in der Einführungsposition (E) lösbar feststellbar ist, insbesondere in der Einführungsposition (E) lösbar feststellbar und einseitig axial gelagert ist.

10. Prozessbehälter (8), insbesondere zur Züchtung von Zellkulturen oder Mikroorgansimen in einem Medium (9),
mit zumindest einem Anschlusselement (6) mit angeordnetem Befestigungselement (14) gemäß einem der vorangegangenen Ansprüche wobei,
das Anschlusselement (6) des Prozessbehälters (8) durch die Anordnung des Befestigungselements (14) an das Anschlusselement (8) fluiddicht abgedichtet ist, insbesondere derart, dass das Messfenster (5) an einen Innenraum des Prozessbehälters (8) angrenzt.

11. Prozessbehälter (8) nach Anspruch 10,
**dadurch gekennzeichnet,**
**dass** das Anschlusselement (6) durch einen Flansch mit einem angeformten Adapter (4) ausbildet ist, bevorzugt mit einem angeformten Schlauchadapter (4), insbesondere mit einem angeformten rohrförmigen Adapter (4), insbesondere bevorzugt mit einem angeformten rohrförmigen Adapter (4) welcher eine kreiszylindrische Innenseite aufweist und außen einen kegelstumpfförmigen Bereich aufweist, besonders bevorzugt mit einem angeformten rohrförmigen Adapter (4) welcher eine kreiszylindrische Innenseite aufweist und außen mehrere axiale Bereiche mit sich unterscheidenden Außendurchmessern aufweist.

12. Prozessbehälter (8) nach einem der Ansprüche 10 bis 11,
**dadurch gekennzeichnet,**
**dass** das Material des Anschlusselements (6) biokompatibel ist, insbesondere aus Polyethylen (PE) ist, bevorzugt aus Polyaryletherketon (PAEK) ist, insbesondere bevorzugt aus Polyethylenterephthalat (PET) oder aus Polypropylen (PP), aus Polyamid (PA), insbesondere aus Polyetheretherketon (PEEK), bevorzugt aus Polyphenylensulfon (PPSU) ist.

13. Prozessbehälter (8) nach einem der Ansprüche 10 bis 12,
**dadurch gekennzeichnet,**
**dass** das Festlegeelement (2) und das zumindest eine Anschlusselement (6) des Bioreaktors konzentrisch angeordnet sind, insbesondere, dass Festlegeelement (2), Sicherungselement (3) und Anschlusselement (6) konzentrisch angeordnet sind.

14. Prozessbehälter (8) nach einem der Ansprüche 10 bis 13,
**dadurch gekennzeichnet,**
**dass** zwischen dem Festlegeelement (2) und dem Anschlusselement (6) ein Ausweichbereich ausgebildet ist, in welchen der bewegbare Rastbereich des zumindest einen Rastarmes (7) bis zum Erreichen der Sicherungsposition (S) des Sicherungselementes (3) ausweichen kann, insbesondere durch Biegen des Rastarmes (7).

15. Prozessbehälter (8) nach einem der Ansprüche 10 bis 14,
**dadurch gekennzeichnet,**
**dass** in den Stutzen (1) des Befestigungselements 14 ein Sensor (10) angeordnet ist und
**dass** der Sensor (10) als optoelektronischer Sensor (10) ausgebildet ist, insbesondere als Trübheitssensor ausgebildet ist.

16. Prozessbehälter nach Anspruch 15,
**dadurch gekennzeichnet,**
**dass** das Befestigungselement (14) gemäß Anspruch 2 ausgebildet ist und dass der Sensor (10) mittels der Kupplung des Stutzens (1) an dem Befestigungselement (14) angeordnet ist, insbesondere über eine Kupplungsdose des Sensors (10), bevorzug eine Schnellverschlusskupplungsdose des Sensors (10).

## Claims

1. Fastening element for arrangement on a connecting element (6) of a process container (8), in particular a reactor, preferably a bioreactor, most preferably a single-use bioreactor,
wherein the fastening element has a nozzle (1) and a fixing element (2),
wherein the nozzle (1) has a sensor opening for inserting a sensor into the nozzle (1) and a measuring window (5) on a side opposite the sensor opening,
and wherein the fixing element (2) is designed to arrange the nozzle (1) on the connecting element (6) of the process container (8),
**characterized in that**
the fixing element (2) has at least one latching arm (7) with a movable latching region in order to form a latching connection with the connecting element (6) of the process container (8),
and the fixing element (2) has at least one securing element (3) arranged displaceably on the fixing element (2), wherein the securing element (3) and the fixing element (2) are designed to interact in such a way that, when the securing element (3) is in an insertion position (E), the latching region of the at least one latching arm (7) is movable and, when the securing element (3) is in a securing position (S), a movement of the latching region of the at least one latching arm (7) is prevented by the securing element (3).

2. Fastening element according to claim 1,
**characterized in that**
the fastening element (2) has a preferably cylindrical base body which is divided into at least two latching arms (7) by preferably axially extending slots.

3. Fastening element (14) according to claim 1 and 2,
**characterized in that**
the securing element (3) is annular in shape and in the securing position encloses the fixing element at least in the latching region.

4. Fastening element (14) according to claim 1, claim 2 and claim 3,
**characterized in that**
the securing element (3) and the fixing element (2) are designed to interact in such a way that the securing element (3) and the fixing element (2) form a positively engaging latching connection in the securing position (S).

5. Fastening element (14) according to claim 1,
**characterized in that**
the measuring window (5) is arranged hermetically sealed against the nozzle (1), in particular via a socket located inside the nozzle (1), preferably a stepped socket.

6. Fastening element (14) according to claim 1,
**characterized in that**
the material of the nozzle (1) is biocompatible, in particular made of polyethylene (PE), preferably of polyaryletherketone (PAEK), particularly preferably of polyethylene terephthalate (PET) or of polypropylene (PP), of polyamide (PA), in particular of polyetheretherketone (PEEK), preferably of polyphenylene sulfone (PPSU).

7. Fastening element (14) according to claim 1,
**characterized in that**
a coupling, preferably a coupling plug, in particular a quick-release coupling plug for arranging a sensor (10), is designed in a region of the sensor opening of the nozzle (1).

8. Fastening element (14) according to claim 1,
**characterized in that**
the material of the measuring window (5) is transparent, preferably made of glass, in particular of glass-ceramic, most preferably of sapphire glass.

9. Fastening element (14) according to claim 1,
**characterized in that**
the securing element (2) can be releasably locked in the insertion position (E), in particular can be releasably locked and is axially mounted on one side in the insertion position (E).

10. Process container (8), in particular for cultivating cell cultures or microorganisms in a medium (9),
with at least one connecting element (6) with an arranged fastening element (14) according to any one of the preceding claims, wherein
the connecting element (6) of the process container (8) is sealed in a fluid-tight manner by the arrangement of the fastening element (14) on the connecting element (8), in particular in such a way that the measuring window (5) adjoins an interior space of the process container (8).

11. Process container (8) according to claim 10,
**characterized in that**
the connecting element (6) is formed by a flange with a moulded-on adapter (4), preferably with a moulded-on hose adapter (4),
in particular with a moulded-on tubular adapter (4), particularly preferably with a moulded-on tubular adapter (4) which has a circular cylindrical inner side and has a frustoconical region on the outside, most preferably with a moulded-on tubular adapter (4) which has a circular cylindrical inner side and has multiple axial regions with differing outer diameters on the outside.

12. Process container (8) according to any one of claims 10 to 11,
**characterized in that**
the material of the connecting element (6) is biocompatible, in particular made of polyethylene (PE), preferably of polyaryletherketone (PAEK), particularly preferably of polyethylene terephthalate (PET) or of polypropylene (PP), of polyamide (PA), in particular of polyetheretherketone (PEEK), preferably of polyphenylene sulfone (PPSU).

13. Process container (8) according to any one of claims 10 to 12,
**characterized in that**
the fixing element (2) and the at least one connecting element (6) of the bioreactor are arranged concentrically, in particular **in that** the fixing element (2), securing element (3) and connecting element (6) are arranged concentrically.

14. Process container (8) according to any one of claims 10 to 13,
**characterized in that**
between the fixing element (2) and the connecting element (6), a deviation region is designed into which the movable latching region of the at least one latching arm (7) can deviate until the securing position (S) of the securing element (3) is reached, in particular by bending the latching arm (7).

15. Process container (8) according to any one of claims 10 to 14,
**characterized in that**
a sensor (10) is arranged in the nozzle (1) of the fastening element (14) and
the sensor (10) is designed as an optoelectronic sensor (10), in particular designed as a turbidity sensor.

16. Process container according to claim 15,
**characterized in that**
the fastening element (14) is designed according to claim 2 and the sensor (10) is arranged on the fastening element (14) by means of the coupling of the nozzle (1), in particular via a coupling socket of the sensor (10), preferably a quick-release coupling socket of the sensor (10).

## Revendications

1. Élément de fixation destiné à être disposé sur un élément de raccordement (6) d'un récipient de processus (8), en particulier d'un réacteur, de préférence d'un bioréacteur, le plus préférentiellement d'un bioréacteur à usage unique,
dans lequel l'élément de fixation présente une tubulure (1) et un élément de fixation (2),
dans lequel la tubulure (1) présente une ouverture de capteur pour l'introduction d'un capteur dans la tubulure (1) et une fenêtre de mesure (5) sur un côté opposé à l'ouverture de capteur,
et dans lequel l'élément de fixation (2) est conçu pour disposer la tubulure (1) sur l'élément de raccordement (6) du récipient de processus (8),
**caractérisé en ce que**
l'élément de fixation (2) présente au moins un bras d'encliquetage (7) avec une zone d'encliquetage mobile pour former une connexion par encliquetage avec l'élément de raccordement (6) du récipient de processus (8)
et l'élément de fixation (2) présente au moins un élément de sécurisation (3) disposé de manière déplaçable sur l'élément de fixation (2), dans lequel l'élément de sécurisation (3) et l'élément de fixation (2) sont conçus pour coopérer de sorte que, dans une position d'introduction (E) de l'élément de sécurisation (3), la zone d'encliquetage de l'au moins un bras d'encliquetage (7) soit mobile et, dans une position de sécurisation (S) de l'élément de sécurisation (3), un mouvement de la zone d'encliquetage de l'au moins un bras d'encliquetage (7) soit empêché par l'élément de sécurisation (3).

2. Élément de fixation selon la revendication 1,
**caractérisé en ce que**
l'élément de fixation (2) présente un corps de base de préférence cylindrique, qui est divisé en au moins deux bras d'encliquetage (7) par des fentes s'étendant de préférence axialement.

3. Élément de fixation (14) selon la revendication 1 et 2,
**caractérisé en ce que**
l'élément de sécurisation (3) est conçu de forme annulaire et renferme l'élément de fixation au moins dans la zone d'encliquetage dans la position de sécurisation.

4. Élément de fixation (14) selon la revendication 1, la revendication 2 et la revendication 3,
**caractérisé en ce que**
l'élément de sécurisation (3) et l'élément de fixation (2) sont conçus pour coopérer de sorte que l'élément de sécurisation (3) et l'élément de fixation (2) forment une liaison à encliquetage s'engageant l'un dans l'autre par complémentarité de formes dans la position de sécurisation (S).

5. Élément de fixation (14) selon la revendication 1,
**caractérisé en ce que**
la fenêtre de mesure (5) est disposée de manière hermétiquement étanche sur la tubulure (1), en particulier par l'intermédiaire d'une monture située à l'intérieur de la tubulure (1), de préférence une monture étagée.

6. Élément de fixation (14) selon la revendication 1,
**caractérisé en ce que**
le matériau de la tubulure (1) est biocompatible, en particulier en polyéthylène (PE), de préférence en polyaryléthercétone (PAEK), en particulier de préférence en polyéthylène téréphtalate (PET) ou en polypropylène (PP), en polyamide (PA), en particulier en polyétheréthercétone (PEEK), de préférence en polyphénylène sulfone (PPSU).

7. Élément de fixation (14) selon la revendication 1,
**caractérisé en ce que**
un accouplement, de préférence une fiche d'accouplement, en particulier une fiche d'accouplement à fermeture rapide, est formé dans une zone de l'ouverture de capteur de la tubulure (1) pour disposer un capteur (10).

8. Élément de fixation (14) selon la revendication 1,
**caractérisé en ce que**
le matériau de la fenêtre de mesure (5) est transparent, de préférence en verre, en particulier en vitrocéramique, le plus préférentiellement en verre saphir.

9. Élément de fixation (14) selon la revendication 1,
**caractérisé en ce que**
l'élément de sécurisation (2) peut être fixé de manière amovible dans la position d'introduction (E), en particulier peut être fixé de manière amovible dans la position d'introduction (E) et logé axialement d'un côté.

10. Récipient de processus (8), en particulier pour la culture de cultures cellulaires ou de microorganismes dans un milieu (9),
avec au moins un élément de raccordement (6) avec un élément de fixation (14) disposé selon l'une quelconque des revendications précédentes, dans lequel,
l'élément de raccordement (6) du récipient de processus (8) est étanchéifié de manière étanche aux fluides par l'agencement de l'élément de fixation (14) sur l'élément de raccordement (8), en particulier de sorte que la fenêtre de mesure (5) soit adjacente à un espace intérieur du récipient de processus (8).

11. Récipient de processus (8) selon la revendication 10,
**caractérisé en ce que**
l'élément de raccordement (6) est formé par une bride avec un adaptateur moulé (4), de préférence avec un adaptateur de tuyau moulé (4),
en particulier avec un adaptateur tubulaire moulé (4), en particulier de préférence avec un adaptateur tubulaire moulé (4) qui présente un côté intérieur cylindrique circulaire et présente à l'extérieur une zone tronconique, le plus préférentiellement avec un adaptateur tubulaire moulé (4) qui présente un côté intérieur cylindrique circulaire et présente à l'extérieur plusieurs zones axiales avec des diamètres extérieurs différents.

12. Récipient de processus (8) selon l'une quelconque des revendications 10 à 11,
**caractérisé en ce que**
le matériau de l'élément de raccordement (6) est biocompatible, en particulier en polyéthylène (PE), de préférence en polyaryléthercétone (PAEK), en particulier de préférence en polyéthylène téréphtalate (PET) ou en polypropylène (PP), en polyamide (PA), en particulier en polyétheréthercétone (PEEK), de préférence en polyphénylène sulfone (PPSU).

13. Récipient de processus (8) selon l'une quelconque des revendications 10 à 12,
**caractérisé en ce que**
l'élément de fixation (2) et le au moins un élément de raccordement (6) du bioréacteur sont disposés concentriquement, en particulier l'élément de fixation (2), l'élément de sécurisation (3) et l'élément de raccordement (6) sont disposés de manière concentrique.

14. Récipient de processus (8) selon l'une quelconque des revendications 10 à 13,
**caractérisé en ce que**
une zone de déviation est formée entre l'élément de fixation (2) et l'élément de raccordement (6) dans laquelle la zone d'encliquetage mobile de l'au moins un bras d'encliquetage (7) peut dévier jusqu'à ce que la position de sécurisation (S) de l'élément de sécurisation (3) soit atteinte, en particulier en pliant le bras d'encliquetage (7).

15. Récipient de processus (8) selon l'une quelconque des revendications 10 à 14,
**caractérisé en ce que**
un capteur (10) est disposé dans la tubulure (1) de l'élément de fixation (14), et
le capteur (10) est conçu en tant que capteur optoélectronique (10), en particulier en tant que capteur de turbidité.

16. Récipient de processus selon la revendication 15,
**caractérisé en ce que**
l'élément de fixation (14) est conçu selon la revendication 2 et le capteur (10) est disposé sur l'élément de fixation (14) au moyen de l'accouplement de la tubulure (1), en particulier par l'intermédiaire d'une prise d'accouplement du capteur (10), de préférence une prise d'accouplement à fermeture rapide du capteur (10).
